# EUROPEAN PATENT APPLICATION

(11) **EP 4 523 686 A1**
(43) Date of publication of application: **19.03.2025**
(21) Application number: 23306533.3
(22) Date of filing: 15.09.2023
(51) Int. Cl.: A61K 31/439, A61P 25/28, A61P 3/00, A61P 21/00, A61P 25/02, A61P 25/08, A61P 25/14, A61P 25/16, A61P 25/18, A61P 25/22, A61P 25/24

(54) **A SIGMA-1 RECEPTOR POSITIVE MODULATOR FOR ITS USE IN THE PREVENTION AND THE TREATMENT OF A PATHOLOGY**

(71) Applicant: UNIVERSITE DE MONTPELLIER, 34090 Montpellier (FR)
(72) Inventor: BOURGUIGNON, Jean-Jacques, 67400 ILLKIRCH-GRAFFENSTADEN (FR); SCHMITT, Martine, 6700 STRASBOURG (FR); MAURICE, Tangui, 34980 SAINT-GÉLY-DU-FESC (FR); DELPRAT, Benjamin, 34830 JACOU (FR); MEUNIER, Johann, 34130 MAUGUIO (FR); CROUZIER, Lucie, 34090 MONTPELLIER (FR)
(74) Representative: IPAZ

(57) **Abstract**

The present invention pertains to a convolamine for its use in the prevention or the treatment of a pathology for its use in the prevention or the treatment of a condition or illness chosen among: a neuropathy, a neurodegenerative pathology, a neurodevelopmental pathology, a psychiatric disorder.

## Description

### FIELD OF THE INVENTION

The present invention pertains to convolamine, or a pharmacologically acceptable salt thereof, for its use in the prevention or the treatment of a neuropathy, a neurodegenerative pathology, a neurodevelopmental pathology, a psychiatric disorder or a metabolic disorder. The present invention therefore concerns the technical domain of pharmacology and more particularly the pharmacology of neuropathologies.

### TECHNICAL BACKGROUND

The Sigma receptor 1, also designated as SIG-1R (SIGMAR1 gene; HGNC: 8157 NCBI Entrez Gene: 10280 Ensembl: ENSG00000147955 OMIM^{®}: 601978 UniProtKB/Swiss-Prot: Q99720) is an intracellular protein mainly distributed, in mammals, on the endoplasmic reticulum membrane and particularly concentrated at the ER contacts with the mitochondria. The 223 amino acid sequence of human SIG-1R (NP_005857.1), and the nucleotide sequences encoding said protein (NM_005866.4) are publicly disclosed.

Physiological functions of SIG-1R comprise, but are not limited to, modulation of Ca²⁺ transfer among intracellular organelles (T. Hayashi *et al.,* 2000), dissociation from the endoplasmic reticulum (ER) stress sensor protein BiP/GRP78 and activation of the IRE-1-dependent and ATF6-dependent ER stress pathways (T. Hayashi & T.P. Su, 2007), potentiation of glutamatergic neurotransmission, potentiation of monaminergic neurotransmissions, increased release of neurotrophic factors particularly brain-derived neurotrophic factor (BDNF), regulation of cellular excitability and PM channels and cholesterol lipid scaffolding.

Since the identification of SIG-1R in the 1990s, some molecules are known to bind orthosterically to SIG-1R, they belong to the group of SIG-1R agonists, and some other molecules are known to bind non-orthosterically to SIG-1R. Molecules that bind non-orthosterically to SIG-1R rather behave as allosteric-type modulators, according to the historical example of phenytoin (Quirion *et al.,* 1992).

SIG-1R agonists, which are molecules capable of activating the chaperone function of the protein, are cytoprotective, anti-amnesic, antidepressant and anticonvulsant, among other functions.

SIG-1R agonists can be developed in indications related to impaired ER-mitochondria communication. Applications of SIG-1R agonists goes therefore beyond the neuroprotective effects described so far in the scientific literature and concerning neurodegenerative diseases such as Alzheimer's disease (AD), Parkinson's disease, Huntington's disease, Sclerosis, etc., and concern in particular indications related to forms of diabetes, mitochondrial diseases and alterations linked to aging.

US 2017/360 798 A1 *"ANAVEX2-73 and certain anticholinesterase inhibitors composition and method for neuroprotection"* discloses that SIG-1R receptor activation is linked to neuroprotection against diseases such as Alzheimer's disease.

WO 2020/013 108 discloses a pharmaceutical composition for preventing or treating mitochondrial diseases, wherein said composition contains a SIG-1R agonist. Mitochondrial diseases are hereditary diseases that result in dysfunction of mitochondrial energy production and are caused by mutation of mitochondrial DNA or nuclear DNA encoding a protein involved in the mitochondrial electron transport system.

WO 2017/191034 discloses a SIG-1R modulator compound for treating neurodegenerative diseases, including Alzheimer's Disease (AD).

EP 2 335 688 « Pharmaceutical compositions comprising sigma receptor ligands », discloses the use of ligands of SIG-1R for treating psychosis and diseases such as Huntington disease and Parkinson disease.

WO 2013/019901 discloses tropinol esters and related compounds to promote the normal processing of amyloid precursor protein (APP). These compounds are described as useful for prophylactic and therapeutic treatment of Alzheimer's disease.

Due to the essential and polyvalent physiological role of SIG-1R and to its implication in many diseases and conditions, there is still a need for ligands of SIG-1R.

The inventors surprisingly demonstrated that convolamine, a tropane alkaloid extracted from *Convulvus subhirsutus* or *Convulvus Plauricalis,* active ingredient of Shankhapushpi, the active principle of Ayurvedic medicine (Shalavadi *et al.,* 2020), is acting as a positive modulator of SIG-1R. This characterization was achieved in well-recognized and specific *in vitro* and *in vivo* test models. Indeed, the molecule appeared as a very effective ligand of Sig-1R in *in vitro* and *in vivo* tests.

Shalavadi *et al.,* 2020, disclosed that *Convolvulus pluricaulis* has potent neuroprotective activity against cerebral ischemic reperfusion injury induced by bilateral common carotid artery occlusion.

Indeed, the inventors identified convolamine as a very effective molecule in a wfs1abKO zebrafish hypermobility test, previously reported to relate to SIG-1R activity (Crouzier et al., 2022).

However, Convolamine did not inhibit the binding of SIG-1R radio-ligand and did not induce the dissociation of the SIG-1R /BiP complex in SIG-1R overexpressing CHO cells, which is a reference SIG-1R activity test. It therefore has no SIG-1R "agonist" activity.

Furthermore, the inventors demonstrated that convolamine facilitated the dissociating effect of the prototype SIG-1R ligand PRE-084 from SIG-1R, in a concentration-dependent manner with an IC₅₀ of 290 nM. Convolamine has therefore a very effective SIG-1R "positive modulator" profile. In this respect, i) its effect on the hyperlocomotion of wfs1abKO zebrafish, ii) its anti-amnesic effect in the model of amnesia induced by dizocilpine in mice and iii) its neuroprotective effect in the pharmacological model of Alzheimer's disease induced by intracerebral injection of amyloid-β25-35 peptide, have been characterized and are all blocked by a selective SIG-1R antagonist, NE-100.

Indeed, convolamine completely prevents the hypermobility of wfs1abKO zebrafish without affecting the mobility of wfs1abWT control fish. It does not displace the SIG-1R radioligand, but rather has a moderate SIG-2R affinity (IC₅₀ = 13 µM). The *in vitro* activity test shows that convolamine alone does not allow the dissociation of SIG-1R and BiP, on the other hand, added at fixed concentrations, it facilitates the dissociation of PRE-084 and SIG-1R, shifting the IC₅₀ of its effect towards higher values. It therefore acts as a positive SIG-1R modulator. The SIG-1R allosteric character of convolamine is strongly suggested by the displacement of the IC₅₀ of PRE-084.

The advantages of the present invention reside in the furniture of an SIG-1R modulator which can be used for preventing or treating conditions or illnesses, in particular for medical and veterinary applications.

Like the known SIG-1R positive modulators (for review, see Vavers et al., 2019), convolamine exhibits an SIG-1R activity *in vivo* giving it potential as an anti-amnestic, antidepressant, anti-epileptic, antioxidant and neuroprotector in various neurodegenerative and ischemic diseases. Its pharmacological interest is that it behaves *in vivo* like agonists with comparable efficacy in terms of intensity of effects and active dose, but does not impact activity under physiological conditions. Modulators are considered to almost devoid of deleterious side effects, unlike orthosteric agonists, and in the case of SIG-1R, a protein itself modulator of cellular pathways, this implies an expected total absence of deleterious side effects.

By analogy with what is known from other positive SIG-1R modulators, convolamine can be defined as an anti-amnesic against the learning deficits induced in mice by dizocilpine and neuroprotective in the pharmacological model of Alzheimer's induced by intracerebral injection of peptide amyloid-β25-35. All of the effects are blocked by co-treatment with NE-100, the reference SIG-1R antagonist, which confirms the efficacy of the SIG-1R activity *in vivo* of convolamine.

A first pharmacological screen on 47 receptors and binding sites only revealed a partial inhibition, at 1 µM, of binding on the 5-HT3 receptor. Convolamine is therefore, at sub-micromolar concentrations, a selective SIG-1R modulator. Furthermore, the molecule could also act as a 5-HT3 antagonist, a potentially additional activity to its SIG-1R component on cognitive and protective effects.

This demonstration opens up possibilities for enhancing the molecule in stabilizing or strengthening cognitive functions, mood and aging of the central nervous system. Since SIG-1R is also highly expressed in the liver, heart, lungs, gonads, peripheral beneficial effects are also expected.

To date, there is no equivalent treatment in the targeted indications. SIG-1R agonist molecules are in clinical development, but they are organic NECs. Apart from the herbal preparations of St. John's wort (which is positioned as acting on SIG-1R in the high micromolar range), there is no commercial supplement with a health indication.

### SUMMARY OF THE INVENTION

In a first aspect, the present invention provides convolamine, or a pharmacologically acceptable salt thereof, for its use in the prevention or the treatment of a condition or illness chosen among: a neuropathy, a neurodegenerative pathology, a neurodevelopmental pathology, a psychiatric disorder and a metabolic disorder.

In a second aspect, the present invention provides a pharmaceutical composition comprising, or consisting of, as active principal ingredient, convolamine or a pharmacologically acceptable salt thereof, and an acceptable carrier, diluent or excipient, for its use in the prevention or the treatment of a condition or illness chosen among: a neuropathy, a neurodegenerative pathology, a neurodevelopmental pathology, a psychiatric disorder and a metabolic disorder.

The present invention also provides a method for the prevention or the treatment of a condition or illness chosen among: a neuropathy, a neurodegenerative pathology a psychiatric disorder and a metabolic disorder, said method comprising the administration of an effective dose of convolamine, or a pharmacologically acceptable salt thereof, to a patient in need thereof.

Characteristics, advantages and uses of the subject matter of the present invention are more detailed hereunder, in an illustrated and non-limiting way. When present, the disclosure of the ranges expressed as "from ... to ..." mean that the limits are included in said ranges.

### Legends to the figures

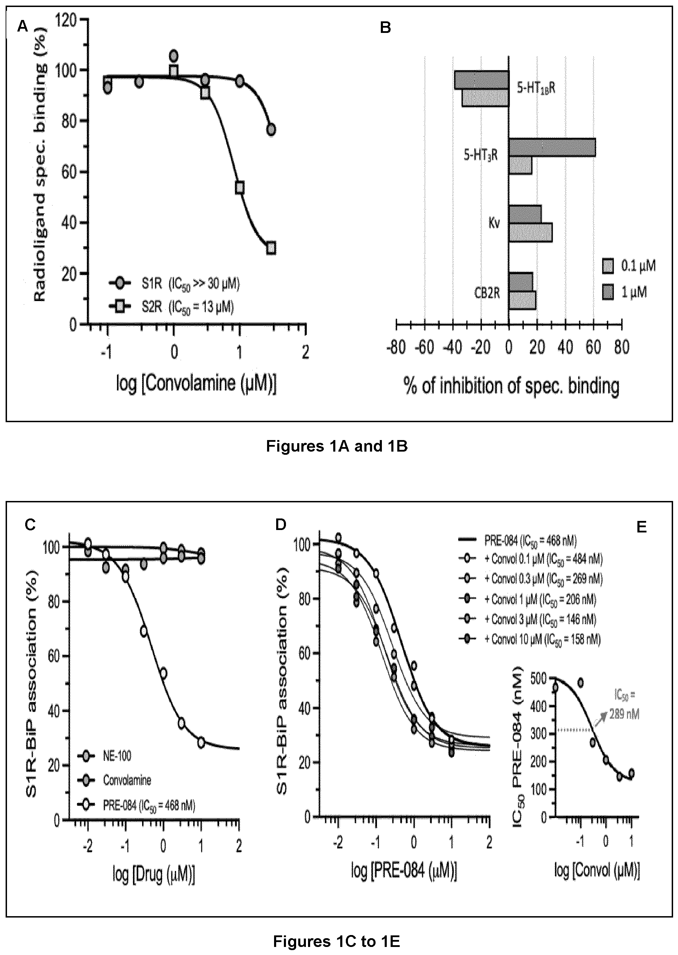

Figures 1A to 1E illustrates the characterisation of convolamine as a SIG-1R positive modulator. Fig. 1A: Dose-response effect of convolamine on inhibition of specific ³H-radioligand binding to SIG-1R (circles) and SIG-2R (square) receptors. Estimated IC₅₀ are indicated in the graph. Fig. 1B: Histogram showing the percentage of inhibition of specific binding in the presence of convolamine. The *in vitro* pharmacological screening on 47 receptors and binding sites showed only marked inhibition of convolamine on CB2 receptors, Kv channels, 5-HT₃ receptor binding and stimulation of 5-HT_{1B} receptors, at 0.1 or 1 µM. Fig. 1C: SIG-1R/BiP dissociation assay for PRE-084 (estimated IC₅₀ of 405 nM), NE-100 and convolamine. GFP-SIG-1R-oe CHO cells were incubated with the test compound for 30 min at 37°C before GFP immunoprecipitation. Fig. 1D: Impact of increasing concentrations of convolamine (0.1-10 µM) on PRE-084-induced dissociation of SIG-1R from BiP. Fig. 1E: estimation of convolamine IC₅₀ value for the displacement of PRE-084 efficacy to dissociate SR1 from BiP.

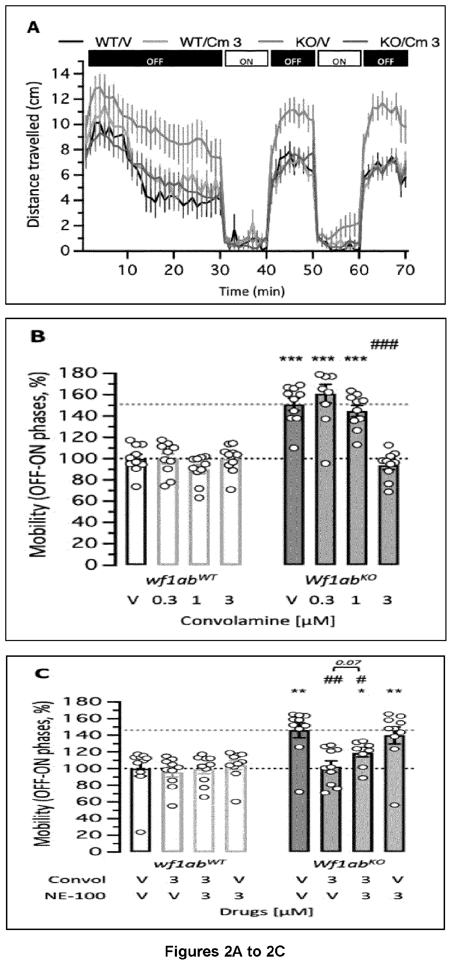

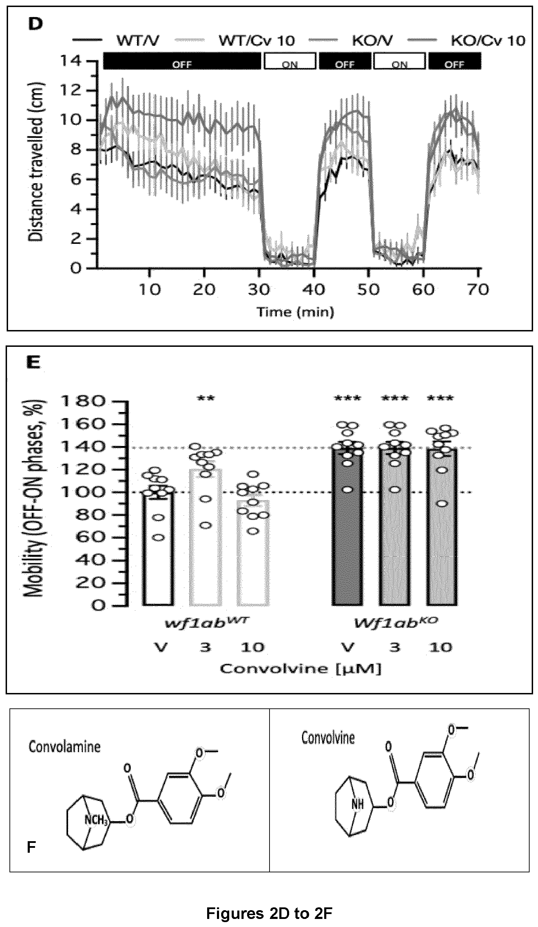

Figures 2A to 2E represent the respective effects of convolamine (Cm) (Fig. 2A to 2C) and of convolvine (Cv) (Fig. 2D and 2E) on the hyperlocomotor response of 5 dpf wfs1ab^{WT} and wfs1ab^{KO} mutant zebrafish larvae. Fig. 2A and 2D: VMR activity profiles. The activity is measured for 70 min, with 30 min of training in the dark (OFF), then 2 cycles of light/dark (ON/OFF) of 10 min each. Fig. 2B, 2C and 2E: Analysis of the distance traveled by 5 dpf wfs1ab^{WT} and wfs1ab^{KO} larvae during the light/dark cycle in the VMR test as averaged differences between the OFF and ON phases. Data show mean ± SEM with n = 8-12 in (Fig.2A, 2B), n = 9-12 in (Fig.2C), n = 11-12 in (Fig. 2D, 2E). Two-way ANOVAs: F_{(1,73)} = 46.04, p < 0.0001 for the genotype, F_{(3,73)} = 17.33, p < 0.0001 for the treatment, F_{(3,73)} = 18.69, p < 0.0001 for the interaction in (B); F_{(1,82)} = 26.21, p < 0.0001 for the genotype, F_{(3,82)} = 19.29, p < 0.0001 for the treatment, F_{(3,82)} = 11.79, p < 0.0001 for the interaction in (C); F_{(1,43)} = 47.94, p < 0.0001 for the genotype, F_{(1,43)} = 21.84, p < 0.0001 for the treatment, F_{(1,43)} = 6.53, p = 0.0021 for the interaction in (E). * p < 0.05, ** p < 0.01, *** p < 0.01 vs. V-treated wfs1ab^{WT} group. unpaired t-test. Fig. 2F represents the respective formulae of convolamine (left panel) and of convolvine (right panel).

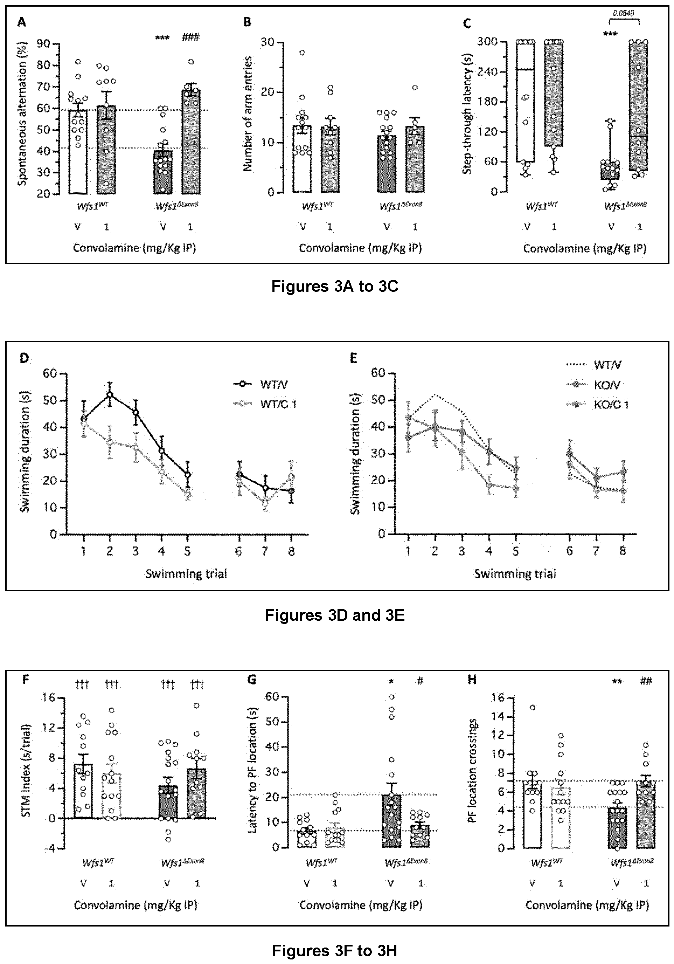

Figures 3A to 3H represent the neuroprotective effect of convolamine on learning impairments in 3-month old *Wfs1^{ΔExon8}* mice: Fig. 3A: spontaneous alternation percentage in the Y-maze; Fig. 3B: number of arm entries in the Y-maze test; Fig. 3C: step-through latency during the passive avoidance retention session; and Fig. 3D to 3H: active avoidance acquisition profiles in the rectangular water-maze, wherein Fig. 3F: short-term memory (STM) index, Fig. 3G: latency to reach the platform location and Fig. 3H: platform location crossings during the retention session. Animals received convolamine (0.3 and 1 mg/Kg IP) 20 min before the Y-maze session or training sessions for the passive and active avoidance tests. Retention was measured 24 h after training for passive avoidance and 72 h after training for active avoidance, without drug injection. Bar graphs show mean ± SEM and individual data in (A, B, F-H) and box-and-whisker graphs show median and interquartile range and individual data in (C). The numbers of animals per groups were: n = 6-15 in (A, B), n = 10-15 in (C), n = 11-17 in (D-H). Two-way ANOVAs: F(1,39) = 1.847, p > 0.05 for the genotype, F(1,39) = 12.87, p = 0.0009 for the treatment, F(1,39) = 9.362, p = 0.0040 for the interaction in (A); F(1,39) = 0.3975, p > 0.05 for the genotype, F(1,39) = 0.2478, p > 0.05 for the treatment, F(1,39) = 0.4856, p > 0.05 for the interaction in (B); F(1,49) = 1.220, p > 0.05 for the genotype, F(1,49) = 0.3765, p > 0.05 for the treatment, F(1,49) = 1.608, p > 0.05 for the interaction in (F); F(1,49) = 5.867, p = 0.0192 for the genotype, F(1,49) = 2.993, p > 0.05 for the treatment, F(1,49) = 4.442, p = 0.0402 for the interaction in (G); F(1,49) = 2.388, p > 0.05 for the genotype, F(1,49) = 2.388, p > 0.05 for the treatment, F(1,49) = 6.633, p = 0.0131 for the interaction in (H). Kruskal-Wallis ANOVA: H = 16.53, p = 0.0009 in (C). * p < 0.05, ** p < 0.01, *** p < 0.001 vs. V-treated *Wfs1^{WT}* group; # p < 0.05, ## p < 0.01, ### p < 0.001 vs. V-treated *Wfs1^{ΔExon8}* group; ††† p < 0.001 vs."0" level; Dunnett's test in (A, B, G, H), Dunn's test in (C), one-column t-test in (F).

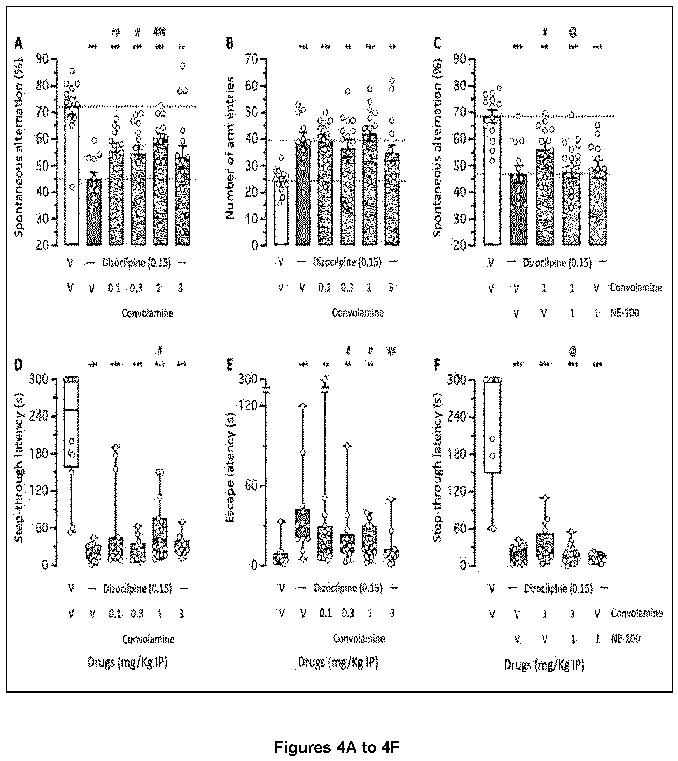

Figures 4A to 4F represent the anti-amnesic effect of Convolamine against Dizocilpine-induced learning impairments in mice. Fig. 4A to 4C: spontaneous alternation percentage in a test in the Y-maze and Fig. 4D to 4F: step-through passive avoidance test. Animals received Convolamine (0.1-3 mg/Kg IP) 10 min before Dizocilpine (0.15 mg/Kg IP), 20 min before the Y-maze test session or passive avoidance training session. Retention was measured 24 h after training. Fig. 4B: number of arm entries in the Y-maze test, Fig. 4D to 4F: step-through latency and Fig. 4E: escape latency. In (C, F) NE-100 (1 mg/Kg) was administered simultaneously as Convolamine (1 mg/Kg IP), 10 min before Dizocilpine. Bar graphs show mean ± SEM and individual data in (A-C) and box-and-whisker graphs and individual data show median and interquartile range in (D-F). The number of animals per groups were: n = 11-16 in (A, B), 12-21 in (C), 11-15 in (D, E), and n = 10-18 in (F). ANOVA: *F*_{(5,79)} = 8.085, *p <* 0.0001, in (A); *F*_{(5,79)} = 5.234, *p* = 0.0003, in (B); *F*_{(4,66)} = 10.73, *p* < 0.0001, in (C). Kruskal-Wallis ANOVA: H = 32.99, *p <* 0.0001, in (D); *H* = 21.1, *p* = 0.0008, in (E); *H* = 29.67, *p <* 0.0001, in (F). ** *p* < 0.01, *** *p* < 0.001 vs. (V+V)-treated group; # *p* < 0.05, ## *p* < 0.01, ### *p <* 0.0001 *vs.* (V+Dizocilpine)-treated group; Dunnett's test in (A-C), Dunn's test in (D-F).

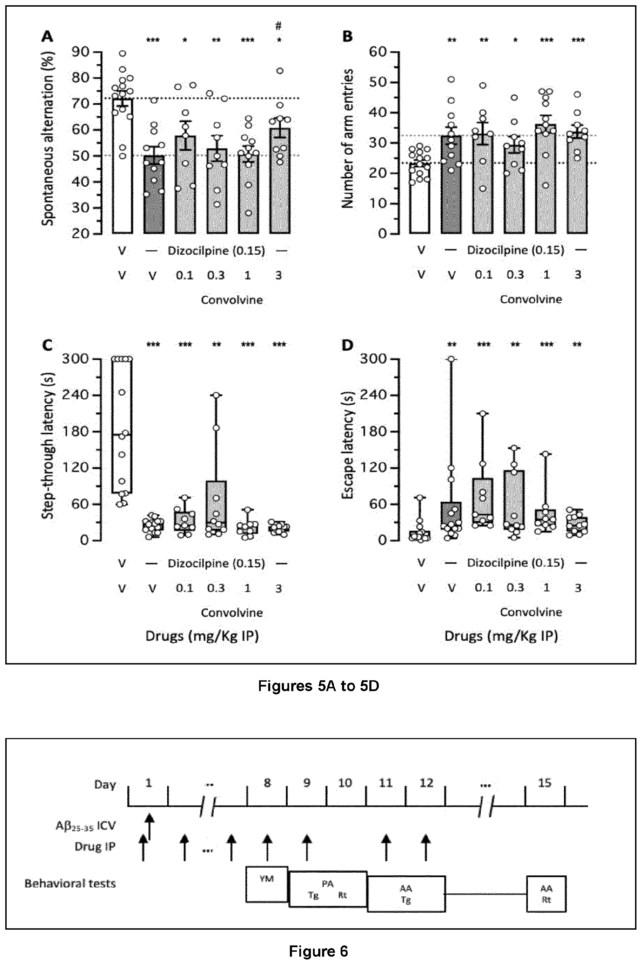

Figures 5A to 5D represent the anti-amnesic effect of Convolvine against Dizocilpine-induced learning impairments in mice: Fig. 5A, 5B: spontaneous alternation test in the Y-maze and Fig. 5C, 5D: step-through passive avoidance test. Animals received Convolvine (0.1-3 mg/Kg IP) 10 min before Dizocilpine (0.15 mg/Kg IP), 20 min before the Y-maze test session or passive avoidance training session. Retention was measured 24 h after training. Fig. 5A: spontaneous alternation percentage, Fig. 5B: number of arm entries in the Y-maze test, Fig. 5C: step-through latency and Fig. 5D: escape latency. Bar graphs show mean ± SEM and individual data in (A, B) and box-and-whisker graphs and individual data show median and interquartile range in (C, D). The number of animals per groups were: n = 9-14 in (A-D). ANOVA: *F*_{(5,56)} = 5.880, p = 0.0002, in (A); *F*_{(5,56)} = 4.002, *p* = 0.0036, in (B). Kruskal-Wallis ANOVA: H = 32.73, *p <* 0.0001, in (C); H = 20.39, *p* = 0.0011, in (D). * *p* < 0.05, ** *p* < 0.01, *** *p <* 0.001 *vs.* (V+V)-treated group; # *p* < 0.05 *vs.* (V+Dizocilpine)-treated group; Dunnett's test in (A-C), Dunn's test in (D-F).
Figure 6 represents the timeline, expressed in days, of the ICV peptide and IP drug injections and of behavioral tests.

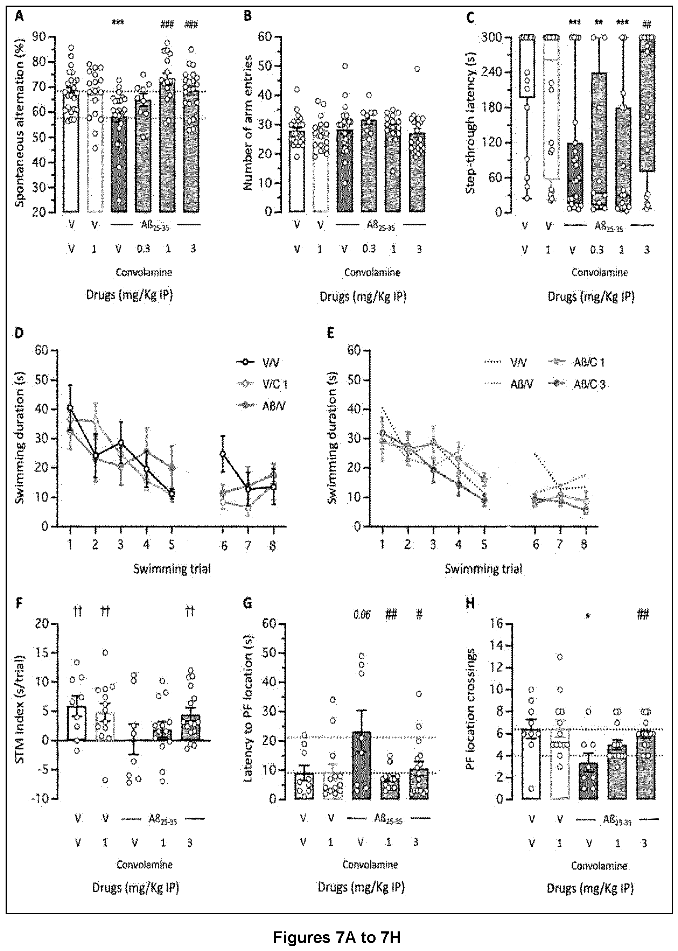

Figures 7A to 7H represent the neuroprotective effect of Convolamine against Aβ₂₅₋₃₅-induced learning impairments in mice. Fig. 7A: spontaneous alternation percentage in the Y-maze. Fig. 7B: number of arm entries in the Y-maze test. Fig. 7C: step-through passive avoidance latency. Fig. 7D to 7H: active avoidance in the rectangular water-maze, with Fig. 7D and 7E: active avoidance acquisition profiles in the rectangular water-maze. Fig. 7F: short-term memory (STM) index, Fig. 7G: latency to to reach the platform location and Fig. 7H: platform location crossings during the retention session. Animals received convolamine (0.3-3 mg/Kg IP) 20 min before Aβ₂₅₋₃₅ peptide (9 nmol ICV) on day 1 and the Y-maze test was performed on day 8, the passive avoidance test on days 9-10 and the active avoidance test on days 11-12, as shown. Retention was measured 24 h after training for passive avoidance and 72 h after training for active avoidance, without drug injection. Bar graphs show mean ± SEM and individual data, except in Fig. 7C, box-and-whisker graphs and individual data show median and interquartile range. The numbers of animals per groups were: n = 5-15 in (A, B), 6-16 in (C-H). ANOVA: *F*_{(5,70)} = 2.954, *p* = 0.0178, in (A); *F*_{(5,70)} = 0.8219, *p* > 0.05, in (B); *F*_{(5,69)} = 2.465, *p* = 0.0412, in (G); *F*_{(5,69)} = 3.247, *p* = 0.0169, in (H). Kruskal-Wallis ANOVA: H = 14.99, *p* = 0.0104, in (C). * *p* < 0.05, ** *p* < 0.01, *** *p <* 0.001 vs. (V+V)-treated group; # *p* < 0.05, ## *p* < 0.01 *vs.* (V+Aβ₂₅₋₃₅)-treated group; ^{†} *p* < 0.05, ^{††} *p <* 0.01, ^{†††} *p <* 0.001 *vs*."0" level; Dunnett's test in (A, B, G, H), Dunn's test in (C), one-column *t*-test in (F).

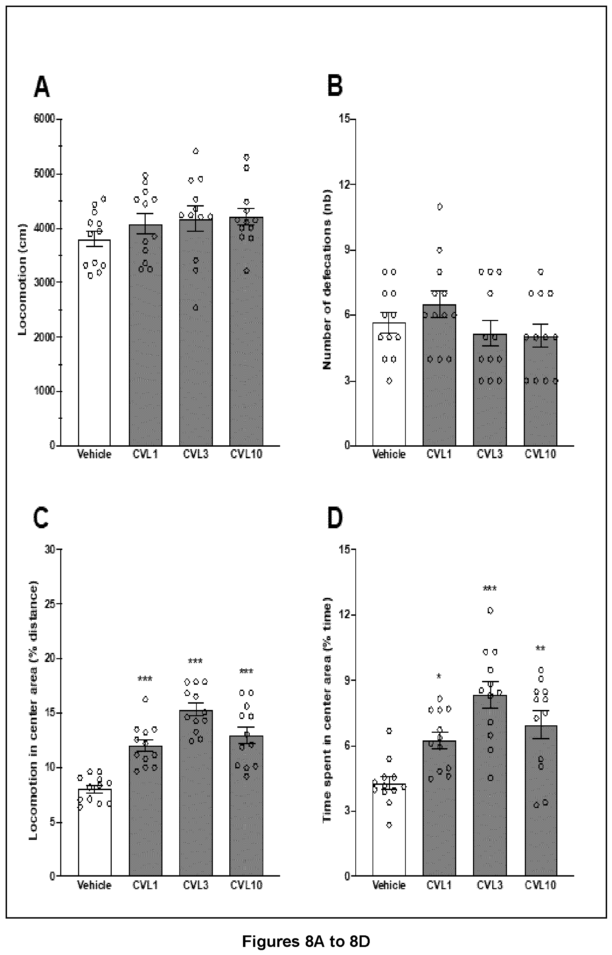

Figures 8A to 8D represent the determination of the anxiolytic effect of convolamine measured by open-field paradigm in mice. In the following conditions: vehicle (no convolamine), CVL1 (1 µM convolamine), CVL3 (3 µM convolamine) and CVL10 (10 µM convolamine), figure 8A represents locomotion (in cm) in the open field, figure 8B represents the number of defecations. figure 8C represents the locomotion in center area (% distance) and figure 8D represents time spent in center area (% time).

### DETAILED DESCRIPTION OF THE INVENTION

In a first aspect, the present invention relates to convolamine, or a pharmacologically acceptable salt thereof, for its use in the prevention or the treatment of a condition or illness chosen among: a neuropathy, a neurodegenerative pathology, a neurodevelopmental pathology, a psychiatric disorder and a metabolic disorder.

The inventors have now characterized convolamine as a positive modulator of SIG-1R.

For the purpose of the invention, "convolamine" is a compound having a molecular chemical formula C₁₇H₂₃NO₄ and the general formula (I):

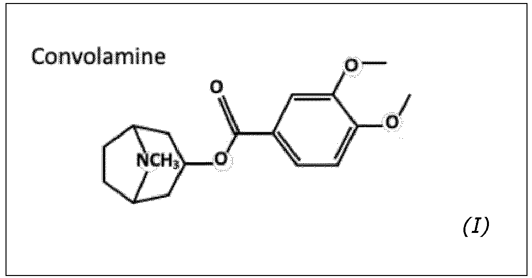

Convolamine is extracted from *convolvulacea* plants (*Convulvus subhirsutus, Convulvus Plauricalis,* Convolvulus *pseudocantabricus*), it belongs to the group of tropanol esters also designated as "tropeins".

An activator of SIG-1R is a ligand of SIG-1R chosen among SIG-1R agonists and SIG-1R positive modulators, wherein the group of said "SIG-1R positive modulators" comprises SIG-1R positive allosteric modulators (PAMs).

By "SIG-1R orthosteric ligands" it is intended a compound that competitively inhibits the specific binding of a radioligand known to slectively bind SIG-1R, including but not limited to [3H](+) SKF-10,047, [3H](+)-pentazocine, [3H]haloperidol in the presence of spiperone, [3H](+)-3-PPP, [3H]-4-IBP and therefore binds to the orthosteric site, also designated as the "active site" of SIG-1R.

By "SIG-1R positive modulator" it is intended a compound that modulates SIG-1R activity by binding SIG-1R at a different site than the orthosteric site. The binding of an allosteric modulator to SIG-1R induces a conformational change in the protein structure and changes the activity of orthosteric ligands.

According to the invention, an activator of SIG-1R is chosen among the group consisting of: selective active modulators of SIG-1R and non-selective active modulators of SIG-1R, such as reviewed by Vavers *et al.* (2019).

Known active modulators of SIG-1R include but are not limited to: Phenytoin (5,5-diphenylimidazolidine-2,4-dione); SKF-83959 (6-chloro-7,8-dihydroxy-3-methyl-1-(3-methylphenyl)-2,3,4,5-tetrahydro-1H-3-benzazepine); methylphenylpiracetam (2-(5-methyl-2-oxo-4-phenyl-pyrrolidin-1-yl)-acetamide, E1R); OZP002 (±)-2-(3-chlorophenyl)-3,3,5,5-tetramethyl-2-oxo-[1,4,2]-oxazaphosphinane); Ropizine ((E)-N-(4-benzhydrylpiperazin-1-yl)-1-(6-methylpyridin-2-yl)methanimine); SKF-38393 ((±)-1-phenyl-2,3,4,5-tetrahydro-(1H)-3-benzazepine-7,8-diol hydrobromide); SCH-23390 (7-chloro-3-methyl-1-phenyl-1,2,4,5-tetrahydro-3-benzazepin-8-ol); SOMCL-668 (3-methyl-phenyl-2, 3, 4, 5-tetrahydro-1H-benzo[d]azepin-7-ol); fenfluramine ((RS)-N-ethyl-1-[3-(trifluoromethyl)phenyl]propan-2-amine).

In the context of the invention, the term "treating" or "treatment", means reversing, alleviating, inhibiting the progress of, or preventing the disorder or condition to which such term applies, or one or more symptoms of such disorder or condition.

In a particular embodiment of this first aspect, the present invention relates to convolamine, or a pharmacologically acceptable salt thereof, for its use in the prevention or the treatment of a neuropathy chosen among: a neuromuscular disorder, or a learning disability.

In the context of the present invention, "a neuromuscular disorder" designates an illness or condition affecting the peripheral nervous system, which consists of all the motor and sensory nerves that connect the brain and spinal cord to the rest of the body. Progressive muscle weakness is the predominant condition of these disorders. This include diabetic neuropathy, amyotrophic lateral sclerosis (Lou Gehrig's disease), Huntington's disease, motor neuron diseases, toxic neuropathy, small fiber neuropathy, autonomic neuropathies, metabolic, neuromuscular junction disorders...

In the context of the present invention, "a learning disability" designates an illness or condition wherein learning and memory processes are impaired, as observed in pathological aging or neuroedegenerative diseases such as mild cognitive impairment, Alzheimer's disease, Parkinson's disease.

In another particular embodiment of this first aspect, the present invention relates to convolamine or a pharmacologically acceptable salt thereof, for its use in the prevention or the treatment of a psychiatric disorder chosen among: psychosis, depression, anxiety, dementia, a memory deficit, amnesia and epilepsy.

In another particular embodiment of this first aspect, the present invention relates to convolamine or a pharmacologically acceptable salt thereof, for its use in the prevention or the treatment of a neurodegenerative pathology chosen among: Alzheimer's disease, Parkinson disease, Huntington disease, amyotrophic lateral sclerosis, multiple sclerosis, and rare neurodegenerative diseases, such as in particular the Wolfram syndrome.

In the context of the present invention, "rare neurodegenerative diseases" designates an illness or condition wherein it affects fewer than 1/2,000 people.

In a more particular embodiment of this first aspect, the present invention relates to convolamine convolamine, or a pharmacologically acceptable salt thereof, for its use in the prevention or the treatment of a human neuropathy, neurodegenerative pathology, neurodevelopmental pathology, psychiatric disorder or metabolic disorder.

In another more particular embodiment of this first aspect, the present invention relates to convolamine or a pharmacologically acceptable salt thereof, for its use in the prevention or the treatment of an animal neuropathy, neurodegenerative pathology, neurodevelopmental pathology, psychiatric disorder or metabolic disorder.

In a second aspect, the present invention relates to a pharmaceutical composition comprising, or consisting of, as active principal ingredient, convolamine or a pharmacologically acceptable salt thereof, and an acceptable carrier, diluent or excipient, for its use in the prevention or the treatment of a condition or illness chosen among: a neuropathy, a neurodegenerative pathology, a neurodevelopmental pathology, a psychiatric disorder and a metabolic disorder.

In a particular embodiment of this second aspect, the present invention relates to a pharmaceutical composition comprising, as active principal ingredient, convolamine or a pharmacologically acceptable salt thereof, and an acceptable carrier, diluent or excipient, wherein convolamine, or a pharmacologically acceptable salt thereof, represents at least 50 %, at least 55 %, at least 60 %, at least 65 %, at least 70 %, at least 75 %, at least 80 %, at least 85 %, at least 90 %, at least 95 %, or 100 % of the active ingredients, expressed as a function of dry weight of the total active ingredients.

A pharmaceutical composition according to the present invention comprises a therapeutically effective amount of convolamine, or a pharmaceutically active salt thereof, as an active modulator of SIG-1R. A "therapeutically effective amount" is intended for a minimal amount of active agent, which is necessary to impart therapeutic benefit to a subject. For example, a "therapeutically effective amount" to a mammal is such an amount, which induces, ameliorates or otherwise causes an improvement in the pathological symptoms, disease progression or physiological conditions associated with or resistance to succumbing to a disorder.

More particularly, the present invention relates to a pharmaceutical composition comprising, as an active ingredient, at least convolamine or a pharmaceutically acceptable salt thereof.

The pharmaceutical composition of the present invention may consist essentially of the active ingredient and at least one physiologically compatible carrier, excipient or diluent.

Examples of dosage forms used for administration of the pharmaceutical composition of the present invention include tablets, granules, powders, capsules, syrups, suspensions, injections, and eye drops. Agents, ointments, patches and the like. These dosage forms can be manufactured using a technique widely used as a usual preparation method, for example, tablets, capsules, oral preparations such as granules, lactose, starch, crystalline cellulose as needed, vegetable oils and other bulking agents, magnesium stearate, lubricants such as talc, binders such as hydroxypropylcellulose and polyvinylpyrrolidone, disintegrators such as calcium carboxymethylcellulose, coating agents such as hydroxypropylcellulose, macrogol and silicone resin. The active ingredient can be formulated using a gelatin coating agent.

For example, a pharmaceutical composition of the invention comprises 0.1 mg, 10 mg, 20 mg, 50 mg, or 100 mg or the like as a dosage unit amount of the active ingredient.

For a method according to the present invention, said administration is, for example, chosen among oral, parenteral, intravenous, intramuscular, intraperitoneal, transdermal, eye drops, intra-auricular or subcutaneous. These administration methods and dosage forms are to be selected according to the patient's condition, age and treatment.

In a particular embodiment of this second aspect, the present invention relates to a pharmaceutical composition, wherein convolamine or a pharmacologically acceptable salt thereof, is present in an amount of at least 80 % of the total dry weight of the composition.

In another particular embodiment of this second aspect, the present invention relates to a pharmaceutical composition, wherein said condition or illness is chosen among: Alzheimer's disease, anxiety and Wolfram syndrome.

In another particular embodiment of this second aspect, the present invention relates to a pharmaceutical composition wherein the therapeutically effective amount of convolamine or a pharmacologically acceptable salt thereof, ranges from about 0.1 to about 10 mg/kg, when administered orally.

In a third aspect, the present invention relates to a method for the prevention or the treatment of a condition or illness chosen among: a neuropathy, a neurodegenerative pathology a psychiatric disorder and a metabolic disorder, said method comprising the administration of an effective dose of convolamine, or any of its salts, to a patient in need thereof.

In a particular embodiment of this third aspect, the present invention relates to a method for the prevention or the treatment of a condition or illness chosen among: a neuropathy, a neurodegenerative pathology a psychiatric disorder and a metabolic disorder, said method comprising the administration of an effective dose of convolamine, or any of its salts, to a patient in need thereof,wherein said patient is a human.

In another particular embodiment of this third aspect, the present invention relates to a method for the prevention or the treatment of a condition or illness chosen among: a neuropathy, a neurodegenerative pathology a psychiatric disorder and a metabolic disorder, said method comprising the administration of an effective dose of convolamine, or any of its salts, to a patient in need thereof, wherein said patient is an animal. In a more particular embodiment of the invention, said animal is a mammal.

### EXAMPLES

### Example 1: Neurological and psychiatric alterations of Wfs1^{ΔExon8} mice

### 1. Materials and methods

### Animals

Male Swiss CD-1 (RjOrl:SWISS) mice (n = 277), aged 7-9 weeks, were supplied from Janvier (Le Genest-Saint-Isle, France) and housed the animal facility of the University of Montpellier (CECEMA, agreement # D34-172-23). *Wfs1^{ΔExon8}* mice were bred in the animal facility of the university of Montpellier (CECEMA) on a 129S6/SvEvTac × C57BL/6J background strain (*Wfs1^{WT}*) by heterozygous crossing. Mice were genotyped by multiplex PCR for both alleles using primers WfsKO_wtF2: 5'-TTGGCTTGTATTTGTCGGCC, NeoR1: 5'-GACCGCTATCAGGACATAGCG and WfsKO_uniR2: 5'-CCCATCCTGCTCTCTGAACC, as previously described (Crouzier *et al.,* 2022). Experiments were performed in 3 months-old littermates. Female and male *Wfs1^{WT}* mice (n = 18 and n = 14, respectively) and female and male *Wfs1^{ΔExon8}* mice (n = 10 and n = 18, respectively) were used in this study. Mice were housed in groups of 5-10 mice, with free access to food and water, in a regulated environment with 23 ± 1°C, 40-60% humidity, and12 h/12 h light/dark cycle. Behavioral testing was performed between 9:00 and 16:00 h.

Zebrafish were purchased at the European Zebrafish Resource Center. Heterozygous *wfs1a^{C799X}* and *wfs1b^{W493X}* mutant lines were maintained in an automated fish tank system (ZebTEC, Tecniplast) at 28°C, pH 7, conductivity around 500 µS and with a 14h:10h light:dark cycle. They were fed with standard diet. Heterozygous *wfs1a*^{*C799X*/*+*} zebrafish were intercrossed to generate wild-type (*wfs1a^{WT}*) and homozygous mutant *wfs1a^{C799X}* zebrafish (*wfs1a^{C799X}*)*,* which became progenitors in order to generate future generations for the experiments. Heterozygous *wfs1b*^{*W493X*/*+*} zebrafish were crossed to generate wild-type (*wfs1b^{WT}*) and homozygous mutant *wfs1b^{W493X}* zebrafish (*wfs1b^{W493X}*)*.* The double mutant line were obtained from crosses of *wfs1a^{C799X}* with *wfs1b^{W493X}* and finally obtained homozygous genotypes *wfs1ab^{WT}* and *wfs1ab^{KO}.* Embryos were collected from natural breeding, raised at 28.5°C in fish water. All animal procedures were conducted in strict adherence to the European Union Directive 2010/63 and ARRIVE guidelines and authorized by the National Ethic Committee (Paris) (APAFIS # 2018051411079082 #15035).

### Drugs and peptides

Dizocilpine and NE-100 were from Sigma-Aldrich (St Quentin Fallavier, France). Convolamine and convolvine were purchased from Latoxan (Porte-lès-Valence, France). They were solubilized in physiological saline and administered intraperitoneally (IP) in mice, in a volume of 100 µl per 20 g body weight.

Amyloid-β[25-35] peptide (Aβ₂₅₋₃₅) was from Eurogentec (Angers, France). It was solubilized in distilled water at 3 mg/ml and stored at -20°C until use. Before injection, the peptide was incubated at 37°C for 4 days, allowing oligomerization. Control injection was performed with vehicle solution (distilled water), and intracerebroventricularly (ICV) injections were done as described previously (Maurice et al., 1996, 2019).

### In vitro binding to SIG-1R and S2R

The assay was performed by CEREP (Celle l'Evescault, France) (study 100062023). SIG-1R binding was performed according to Ganapathy *et al.* (1999). Increasing drug concentrations (100 nM to 30 µM) were incubated with 15 nM of [³H](+)-pentazocine in membranes preparations from Jurkat human leukemic T cells. Samples were incubated during 120 min at 37°C and nonspecific binding level was determined using 10 µM of haloperidol. S2R binding was performed according to Ganapathy *et al.* (1999). Increasing drug concentrations (100 nM to 30 µM) were incubated with 25 nM of [³H]ditolyl-guanidine in membranes preparations from Jurkat human leukemic T cells. Samples were incubated during 60 min at room temperature and nonspecific binding level was determined using 10 µM of haloperidol. Assays were performed in duplicates and results were expressed as % of specific binding level.

### SIG-1R/BiP dissociation assay

The assay was performed as described by Hayashi and Su (2007). CHO cells which overexpress GFP tagged-SIG-1R (gift from Tsung-Ping Su, NIDA/NIH, Baltimore, MD, USA) were maintained in DMEM/Glutamax culture medium supplemented with 10% heat inactivated FBS. GFP-SIG-1Roe CHO cells were plated in 6-well plates and treated with test compounds dissolved in culture medium for 30 min at 37°C. The reaction was terminated by removing the culture medium containing the test drug and replacing it with 1 ml of PBS. GFP-SIG-1R-oe CHO cells were harvested and suspended in PBS (pH 7.4) followed by cross-linking with 50 µg/ml of dithiobis succinimidyl propionate (ThermoFisher, France). The reaction was stopped by adding Tris/HCl 50 mM (pH 8.8). After a 15 min incubation on ice, cells were lysed with 50 mM Tris (pH 7.4), 150 mM NaCl, 1% Triton X/100, 0.3% sodium deoxycholate, 0.1% SDS buffer containing a protease inhibitor cocktail (Sigma-Aldrich, France). After centrifugation at 16,000 g for 1 min, the supernatant was incubated with Chromotek GFP-trap agarose (Proteintech, United Kingdom) overnight at 4°C. After centrifugation at 16,000 g for 1 min, the supernatant was discarded and the pellet was suspended in 0.5 ml buffer (50 mM Tris (pH 7.4), 150 mM NaCl, 1% Triton X/100, 0.3% sodium deoxycholate, 0.1% SDS), rinsed twice, and analyzed by Heat Shock 70 kKDa Protein 5 ELISA assay (#CL-SEC343Mu, Euromedex, France).

### Visual motor response in zebrafish larvae

The visual motor response assay quantifies the locomotor activity of the zebrafish larvae to light intensity changes. The protocol was previously described (Crouzier et al., 2021a, 2021b, 2022). In brief, 5 dpf larvae were transfered in a 96-well plate (#7701-1651, Whatman) with 300 µl of fish water and placed in a Zebrabox^{®} (ViewPoint, Lissieu, France). The locomotor behavior was monitored using an automated videotracking device equipped with an infra-red (IR) camera under IR light illumination (Zebralab^{®}, Viewpoint). The light protocol was as follow: 30 min of acclimatization in the dark (0% light intensity), then 2 cycles of 10-min duration light ON and 10-min duration light OFF periods. The brightness changes were immediate (<< 1 s). The activity was measured in mm/s. For each larva, the mobility were calculated as mean (OFF-ON) values to account for inter- and intra-group variability in locomotor response.

### Spontaneous alternation in the Y-maze in mice

The Y-maze is made of grey polyvinylchloride. Each arm is 40 cm long, 13 cm high, 3 cm wide at the bottom, 10 cm wide at the top, and converging at an equal angle. Each mouse was placed at the end of one arm and allowed to move freely through the maze during an 8 min session. The series of arm entries, including possible returns into the same arm, were checked visually. An alternation is defined as entries into all three arms on consecutive occasions. The number of maximum alternations is therefore the total number of arm entries minus two and the percentage of alternation was calculated as (actual alternations / maximum alternations) × 100 (Maurice *et al.,* 1996; Meunier *et al.,* 2006, 2013). Exclusion criteria are locomotion < 10 or percentage of alternation < 25% or > 90%. Animals showing these readouts are excluded from the calculations. Attrition is routinely < 5% in this test.

### Step-through passive avoidance in mice

The apparatus is a two-compartments (15 × 20 × 15 cm high) box with one illuminated with white polyvinylchloride walls and the other darkened with black polyvinylchloride walls and a grid floor. A guillotine door separates each compartment. A 60 W lamp positioned 40 cm above the apparatus lights up the white compartment during the experiment. Scrambled foot shocks (0.3 mA for 3 s) could be delivered to the grid floor using a shock generator scrambler (Lafayette Instruments, Lafayette, USA). The guillotine door was initially closed during the training session. Each mouse was placed into the white compartment. After 5 s, the door was raised. When the mouse entered the darkened compartment and placed all its paws on the grid floor, the door is closed and the foot shocks delivered for 3 s. The step-through latency (STL-Tg), that is, the latency spent to enter the darkened compartment, and the number of vocalizations were recorded. The retention test was carried out 24 h after training. Each mouse was placed again into the white compartment. After 5 s, the door was raised. The step-through latency (STL-R) was recorded up to 300 s (Meunier et al., 2006, 2013; Villard et al., 2009, 2011). An exclusion criteria was STL-Tg and STL-R < 10 s. Animals showing these readouts are excluded from the calculations. Attrition is routinely < 5% in this test.

### Active avoidance in the rectangular water-maze in mice

Non-spatial long-term memory was also examined using the successive training, place-learning procedure in the rectangular water-maze (Maurice *et al.,* 1995, 2018). The apparatus is a black Plexiglas rectangular pool (30 × 60 × 36 cm high), filled with water at a high of 25 cm. The water temperature was maintained at 23 ± 1°C. A transparent Plexiglas platform (5 × 8.5 × 0.5 cm) was fixed in the southeast corner of the pool, 1 cm below the water surface. On acquisition days 1 and 2, each mouse was placed at the middle of the west side of the pool, facing the wall, and the latency to find the platform was recorded up to 60 s. The mouse was allowed to remain on it for 20 s, and then removed from the pool into its home cage. If the mouse did not find the platform within 60 s, the latency was assigned as 60 s, and it was manually placed on it, left for 20 s, and removed to its home cage. Each animal was run in this manner for a total of 5 trials on day 1, and 3 trials on day 2. The starting location did not change during all training sessions. A short-term memory (STM) index was defined as the mean difference in swimming duration between trial *n* and trial *n-1* for trials 2 to 5 and 7 to 8. The STM index was analysed vs. zero level (= no improvement). On day 4, 48 h after the last training session, animals were tested for retention. The platform was removed. Each mouse was again placed at the starting point and observed for 60 s. The latency to reach the original platform location and the time spent on the platform location was recorded.

### Statistical analyses

Analyses were done using Prism v9.0 (GraphPad Software, San Diego, CA, USA). Data were analyzed using one-way analyses of variance (ANOVA, F value), followed by a Dunnett's test. Passive avoidance latencies, expressed as median and interquartile range and represented as box-and-whiskers, were analyzed using a nonparametric Kruskal-Wallis ANOVA (H value) and *post-hoc* comparisons done using a Dunn's test. STM data were analyzed using a one-sample *t-*test vs. "no-learning" level (0 s). Significance levels were *p* < 0.05, *p <* 0.01 and *p <* 0.001.

### 2. Results

### 2.1. Phenotypic screening demonstrated convolamine as a SIG-1R positive modulator

A phenotypic test described by Crouzier *et al.* (2022) was performed. In brief, *wfs1ab^{KO}* zebrafish exhibited an increased mobility response in the VMR assay as compared *to wfs1ab^{WT}* controls. This hyperlocomotor response is known to be selectively prevented by SIG-1R agonists, the drugs failing to affect the basal locomotor response in *wfs1ab^{WT}* controls. Compounds were added in the fish water of larvae at 4 dpf for 24 h, and screening parameters included: (1) the lack of toxicity (including grossly observable morphological alterations) after 24 h exposure; (2) the locomotor increase in *wfs1ab^{KO}* larvae, expressed as % of V-treated *wfs1ab^{KO}* response; (3) the eventual alteration of locomotion in *wfs1ab^{WT}* larvae, expressed as % of V-treated *wfs1ab^{WT}* response; and (4) the Protection index (PI) calculated as detailed in the Methods section.

Data obtained showed that, at 3 µM, *i.e.,* leading to 0.8 < PI < 1.2, the tropane alkaloid convolamine completely prevented the hyperlocomotor response of *wfs1ab^{KO}* zebrafish without affecting basal mobility in *wfs1ab^{WT}* controls (Fig. 1B, 1C).

Analysis of the SIG-1R binding potency of convolamine revealed no affinity as IC₅₀ for [³H](+)-pentazocine binding was higher than 30 µM (Fig. 1D). Convolamine showed rather a moderate affinity for S2R binding sites, as the IC₅₀ for [³H]DTG binding was about 13 µM (Fig. 1D).

Convolamine was tested in a panel of 47 receptors and ionophores (CEREP) and appeared inactive on all targets except for CB2 receptors, Kv channels and 5-HT₃ receptors, for which 20-60% inhibitions of the selective radiotracer were observed at micromolar concentrations, and 5-HT_{1B} receptors, for which a 30-40% stimulation of specific binding was observed (Fig. 1E).

Table 1 represents pharmacological profiling of convolamine which was tested at 1000 nM and 1 µM in *in vitro* binding assays designed with appropriate reference compounds in duplicates at CEREP facility.

**Table 1**

| *Pharmacological targets* | *Inhibition at 0.1* & *1 µM* |
|---|---|
| 5-HT3 receptor | 16.2% & 61.7% |
| 5-HT1B receptor | -33.0% & -38.6% |
| Kv channel | 30.4% & 22.7% |

SIG-1R activity was analysed using the SIG-1R/BiP dissociation assay (Hayashi & Su, 2007; Maurice *et al.,* 2019) in GFP-SIG-1R-overexpressing CHO cells. The prototypic SIG-1R agonist PRE-084 dissociated SIG-1R from BiP with an IC₅₀ of 468 ± 7 nM, while the SIG-1R antagonist NE-100 was ineffective at concentrations up to 30 µM (Fig. 1E). Convolamine, at concentrations up to 30 µM failed to dissociate SIG-1R from BiP (Fig. 1E), confirming that the drug is not a SIG-1R agonist. However, when convolamine was co-administered at a fixed concentration (0.1-10 µM) with increasing concentrations of PRE-084, the inventors observed a shift in the PRE-084-response curve to lower concentrations and IC₅₀ values decreased to 140-150 µM (Fig. 1F). This demonstrated a SIG-1R positive modulatory action of convolamine, with an IC₅₀ value calculated at 289 nM (Fig. 1G).

These results identified convolamine as a SIG-1R positive modulator, devoid of SIG-1R affinity for the "orthosteric" agonist binding site but able to potentiate the *in vitro* activity of SIG-1R agonist marked in vitro and showing *in vivo* activity in a phenotypic screening test.

### Example 2: Convolamine fully prevented behavioral alterations in wfs1ab^{KO} zebrafish and mouse models

Convolamine was tested in the 0.3-3 µM concentration-range in the visual motor response assay in *wfs1ab^{KO}* zebrafish larvae and dose-dependently abolished the hyperlocomotor response, without affecting basal response in *wfs1ab^{WT}* control larvae (Fig. 2A, 2B). This effect was attenuated by co-treatment with the SIG-1R antagonist NE-100, at 3 µM (Fig. 2C). Indeed the NE-100+Convolamine treated larvae showed a close-to-significance (p = 0.07) increased locomotor response as compared to convolamine-treated larvae. The SIG-1R antagonist failed to affect locomotion in *wfs1ab^{WT}* controls or the hyperlocomotor response in *wfs1ab^{KO}* zebrafish (Fig. 2C).

Convolvine, the desmethyl metabolite of convolamine, was also tested at 3 µM and failed to attenuate the hyperlocomotor response in *wfs1ab^{KO}* zebrafish (Fig. 2D, 2E).

Convolamine was then tested in a mouse model of WS, *Wfs1^{ΔExon8}* mouse line. Male *Wfs1^{ΔExon8}* mice show learning and memory deficits that could be selectively attenuated by SIG-1R agonists (Crouzier, Danese *et al.,* 2022). In the Y-maze test, *Wfs1^{ΔExon8}* mice showed a significant alteration of spontaneous alternation as compared to *Wfs1^{WT}* mice that was significantly attenuated by convolamine at 1 mg/Kg IP (Fig. 3A). The exploratory response was unaltered either by the genotype or by the treatment (Fig. 3B). In the passive avoidance test, the step-though latency was significantly decreased in *Wfs1^{ΔExon8}* mice and convolamine attenuated the deficit (Fig. 3C). Indeed, the latency showed by convolamine- treated mice was not significantly different from control V-treated *Wfs1^{WT}* mice and the improvement as compared with convolamine-treated *Wfs1^{WT}* mice was close-to-significance (*p* = 0.0549; Fig. 3C). In the rectangular water-maze, mice were trained to locate a small rectangular platform opposite to their departure location. All groups showed acquisition profiles that decreased over the 8 training sessions (5 on day 1 and 3 on day 2) as shown in Fig. 3D for Wfs1^{WT} mice and in Fig. 3E for *Wfs1^{ΔExon8}* mice. The calculation of the STM index showed that all groups significantly improved the latency from one trial to the other, but V-treated *Wfs1^{ΔExon8}* mice presented a mild deficit (-3 s vs. V-treated Wfs1^{WT} mice; Fig. 3F), that was not observed after the convolamine treatment. During the probe test, performed without platform, 48 h after the last training session, *Wfs1^{ΔExon8}* mice showed a higher latency to reach the platform location and a lower number of platform location crossings as compared to *Wfs1^{WT}* mice (Fig. 3G, 3H). The convolamine treatment attenuated these differences, as evidenced at least by significant interaction between the two-way ANOVA factors and *post-hoc* significant differences. Therefore, as expected for a SIG-1R acting drug (Crouzier, Danese *et al.,* 2022), convolamine attenuated the memory deficits in *Wfs1^{ΔExon8}* mice.

### Example 3. Convolamine is an anti-amnesic and neuroprotective compound through its SIG-1R activity

Due to the chaperone nature of SIG-1R, a SIG-1R positive modulator behaves as an agonist *in vivo,* particularly on learning and memory abilities in mice (Maurice *et al.,* 2019; Vavers *et al.,* 2019). Convolamine was tested on Dizocilpine-induced learning impairments, a well-characterized SIG-1R-mediated response in mice (Maurice *et al.,* 1994, 2001). The NMDA receptor noncompetitive antagonist Dizocilpine (0.15 mg/kg IP) prevented spontaneous alternation behavior (Fig. 4A) and increased mobility (Fig. 4B) in the Y-maze. The convolamine treatment attenuated, dose-dependently and significantly but in a bell-shaped manner, the Dizocilpine-induced alternation deficit, in the 0.1-1 mg/kg IP range (Fig. 4A). Convolamine however did not affect the hyperlomocomotor response to dizocilpine (Fig. 4B). Co-treatment with the SIG-1R antagonist NE-100 at 1 mg/kg IP, blocked the attenuating effect of Convolamine (Fig. 4C), thus confirming the SIG-1R-mediated effect.

In the passive avoidance test, Dizocilpine significantly decreased the step-though latency (Fig. 4D) and increased the escape latency (Fig. 4E) measured during retention. The convolamine treatment attenuated significantly these alterations, at 1 mg/kg IP for the step-through latency (Fig. 4D) and at 0.3-3 mg/kg IP, for escape latency (Fig. 4E). NE-100 co-treatment also blocked convolamine effect, shown in Fig. 4F for the step-though latency.

Convolvine was also tested against Dizocilpine-induced learning deficits in the same 0.1-3 mg/kg IP dose range. The drug significantly attenuated Dizocilpine-induced alternation at the highest dose tested (Fig. 5A), without affecting Dizocilpine-induced hyperlocomotor response (Fig. 5B). In the passive avoidance, convolvine attenuated, but in a non-significant manner, the decrease in step-through latency at 0.3 mg/kg IP (Fig. 5C) and the increase in escape latency, at the highest doses tested (Fig. 5D). Therefore, convolvine appeared less effective than it related drug convolamine.

SIG-1R acting drugs are potent neuroprotective drugs in several neurodegenerative disease indications. Moreover, Blarcamesine, the drug presently tested in a clinical phase 3 assay (NCT04314934) was identified in the pharmacological model of AD induced in mice by ICV administration of oligomerized Aβ₂₅₋₃₅ peptide (Villard *et al.,* 2011). Convolamine was administered only once, 20 min after the Aβ₂₅₋₃₅ peptide ICV, on day 1, *i.e.,* one week before the behavioral analyses (Fig. 7), according to the protocol described by Meunier *et al.* (2006) for SIG-1R-mediated neuroprotection. Convolamine was tested in the 0.3-3 mg/kg IP dose range and at all doses it significantly attenuated the Aβ₂₅₋₃₅-induced deficit in spontaneous alternation (Fig. 7A). Convolamine was however not effective in the passive avoidance test as it did not attenuate the Aβ₂₅₋₃₅-induced deficit (Fig. 7C). In the active avoidance test, acquisition profiles (Fig. 7D, E) showed that Aβ₂₅₋₃₅-treated animals were impaired. Indeed, the STM showed by Aβ₂₅₋₃₅-treated mice was not significantly different from zero, contrarily to Veh-treated controls (Fig. 6F). The convolamine treatment at 1 mg/kg significantly increased STM. The drug effect was confirmed in the retention session, as the Aβ₂₅₋₃₅-induced increase in latency (Fig. 6G) and decrease in number of platform location crossings (Fig. 6H) were significantly attenuated by convolamine at 1 mg/kg IP.

### Conclusions

In the present data report, the alkaloid convolamine acts as a SIG-1R positive modulator. Convolamine blocks the hyperlocomotion in *wfs1ab^{KO}* mutant zebrafish larvae without affecting spontaneous locomotion in *wfs1ab^{WT}* controls, thus showing a PI of 1.0. This screening assay was designed to identified SIG-1R agonists (Crouzier et al., 2022), however convolamine failed to inhibit [³H](+)-pentazocine binding to SIG-1R at concentrations < 10 µM, thus not acting as a SIG-1R ligand. However, the drug showed a moderate affinity for S2R (IC₅₀ = 13 µM) and some potency to inhibit binding to 5-HT₃ receptor and to enhance binding to 5-HT_{1B} receptor at 1 µM. Analysis of the SIG-1R activity of convolamine showed that the drug failed to dissociate SIG-1R from BiP at concentrations < 10 µM, confirming that the drug is not a SIG-1R agonist, but increased the activity of PRE-084, with an estimated IC₅₀ value of 290 nM. Convolamine acted as a potent, and relatively selective, SIG-1R positive modulator, as previously described for OZP-002 or Fenfluramine (Maurice *et al.,* 2019; Vavers *et al.,* 2019).

A characteristic of SIG-1R positive modulators, likely due to the chaperone nature of SIG-1R, is to behave *in vivo* as agonists (Vavers *et al.,* 2019). Indeed, convolamine (1) prevented the hyperlocomotion of *wfs1ab^{KO}* zebrafish; (2) attenuated in a bell-shaped manner the dizocilpine-induced amnesia; and (3) prevented Aβ₂₅₋₃₅ induced learning deficits when injected once simultaneously as the peptide. The active dose of convolamine was in the 0.3-3 mg/kg dose-range, as observed for PRE-084 in these tests. All these effects were prevented by NE-100, the prototypic SIG-1R antagonist, and convolvine, the desmethyl metabolite of convolamine was also but less active, at higher doses.

In conclusion, the alkaloid convolamine was identified as a potent SIG-1R positive modulator that, *in vivo,* may show plasticity-enhancing, anti-amnesic, antidepressant and neuroprotective activities, as observed for SIG-1R acting drugs.

### Example 4: Convolamine exerts an anxiolytic effect

### 1 Material and methods

### Drug

Informations regarding convolamine: Denomination: Convolamine, Synonym: (8-methyl-8-azabicyclo[3.2.1]octan-3-yl) 3,4-dimethoxybenzoate, CAS n°: 500-56-1, Supplier: Latoxan Laboratory, Reference: L6086, Batch: 313-020, Molecular Weight: 305.37, Storage Temp: RT, Appearance: white powder, Vehicle: Physiological saline 0.9%

Drug administration: Route: intraperitoneal (IP), Method: by injection. The drug was administered under a volume calculated according to the individual body weight of each mouse (5 ml/kg), Frequency: once before the behavioral procedure, Treatment length: once, Timing: 30 minutes before behavioral procedure.

### Animals

Male Swiss mice, weighing 30-35 g, from JANVIER (Saint Berthevin, France), were kept for housing and experiments took place within CECEMA animal facility (Direction Régionale de l'Alimentation, de l'Agriculture et de la Forêt du Languedoc-Roussillon, agreement #E 34-172-23 from September 27, 2022). Animals were housed in groups with access to food and water *ad libitum* (#A04C, Safe Diet, Augy, France). They were kept in a temperature and humidity controlled animal facility on a 12 h/12 h light/dark cycle (lights off at 07:00 pm). Mice were numbered by marking their tail with permanent markers. All animal procedures were conducted in strict adherence to the European Union directive of September 22, 2010 (2010/63/UE).

### Mortality and observation of animals

Health of animals was checked daily: general aspect of animals, activity, acute or delayed mortality. Weight was monitored the day of experiment. No mortality nor adverse effect of compound were observed.

### Euthanasia

After behavioral experiment, animals were euthanized by cervical dislocation.

### Behavioral procedure

The day of the test, animals were tested for general locomotor activity in the open field (OF) activity test for 10 min. The open field is made of opaque white plexiglass. The arena is composed of a large square box (50cm × 50cm × 50cm). Animal movements were followed by an overhead camera placed above the apparatus. Each mouse was placed in the center of the arena and allowed to move freely through the arena during its session. The locomotor activity was assessed by counting the total distance traveled measured in centimeters (cm) for the whole session time. The time spent in center area, and the number of defecations were also assessed.

### Statistical analyses

All values were expressed as mean ± S.E.M. Statistical analyses were performed using one-way ANOVA (F value), followed by Dunnett's post-hoc multiple comparison test. Values with p *<* 0.05 were considered as statistically significant.

### 2 Results

The animal model used is the mouse. The behavioral test used is the measure of physiological anxiety in the elevated plus maze test.

Convolamine injected 30 minutes before the open-field test showed no particular effect in total locomotion (Figure 8A). Convolamine also does not modify the number of defecation in animals compared to vehicle group (Figure 8B).

Convolamine induced an increase of the percentage of locomotion in center of the arena (Figure 8C), and an increase of time spent in the center of the arena (Figure 8D). As rodents have an instinctive propensy to avoid open spaces, a property refer to as thigmotaxis, the will spontaneously spent more time walking along the walls. An anxious behavior will increase the time spent along the walls while an anxiolytic behavior will reuslt in an increase in time spent in the center of the open field. Convolamine therefore showed a significant and bell-shaped increase in time spent in the center compared to the vehicle-treated group, denoting anxiolytic properties in the test.

### BIBLIOGRAPHIC REFERENCES:

Crouzier L, et al. Science Transl Med. 2022;14(631):eabh3763. doi:
10.1126/scitranslmed.abh3763.
Crouzier L, et al. Int J Mol Sci. 2021;22(20):11049. doi: 10.3390/ijms222011049.
Crouzier L, et al. Hum Mol Genet. 2022;ddac065. doi: 10.1093/hmg/ddac065.
Ganapathy ME, et al. J Pharmacol Exp Ther. 1999;289(1):251-60.
Hayashi T, Su TP. Cell. 2007;131(3):596-610. doi: 10.1016/j.cell.2007.08.036.
Hayashi, T. et al., J Pharmacol Exp Ther 2000; 293(3): 788-98.
Maurice, T. et al., Brain Res. 1994a;647:44-56. doi: 10.1016/0006-8993(94)91397-8
Maurice, T. et al., Pharmacol Biochem Behav. 1994b;49:859-69. doi: 10.1016/0091-3057(94)90235-6.
Maurice T, et al., Brain Res. 1996;706(2): 181-93. doi: 10.1016/0006-8993(95)01032-7.
Maurice T, et al., J Neural Transm Gen Sect. 1995; 102(1): 1-18. doi: 10.1007/BF01276561.
Maurice T, et al., Pharmacol Res. 2019;144:315-330. doi: 10.1016/j.phrs.2019.04.026.
Meunier J. et al., Br J Pharmacol. 2006;149(8):998-1012. doi: 10.1038/sj.bjp.0706927
Meunier J, et al. Eur J Pharmacol. 2013; 698(1-3):193-9. doi: 10.1016/j.ejphar.2012.10.033.
Shalavadi et al., J Ethnopharmacol. 2020;249: 112393. doi: 10.1016/j.jep.2019.112393.
Vavers E, et al. Front Pharmacol. 2019;10:223. doi: 10.3389/fphar.2019.00223.

## Claims

1. Convolamine, or a pharmacologically acceptable salt thereof, for its use in the prevention or the treatment of a condition or illness chosen among: a neuropathy, a neurodegenerative pathology, a neurodevelopmental pathology, a psychiatric disorder and a metabolic disorder.

2. Convolamine, or a pharmacologically acceptable salt thereof, according to claim 1, for its use in the prevention or the treatment of a neuropathy chosen among: a neuromuscular disorder, a locomotion deficit and a learning disability.

3. Convolamine, or a pharmacologically acceptable salt thereof, according to claim 1, for its use in the prevention or the treatment of a psychiatric disorder chosen among: psychosis, depression, anxiety, dementia, a memory deficit, amnesia and epilepsy.

4. Convolamine, or a pharmacologically acceptable salt thereof, according to claim 1, for its use in the prevention or the treatment of a neurodegenerative pathology chosen among: Alzheimer's disease, Parkinson disease, Huntington disease, amyotrophic lateral sclerosis, multiple sclerosis, and rare neurodegenerative diseases including the Wolfram Syndrome.

5. Convolamine, or a pharmacologically acceptable salt thereof, according to any of claims 1 to 4, for its use in the prevention or the treatment of a human neuropathy, neurodegenerative pathology, neurodevelopmental pathology, psychiatric disorder or metabolic disorder.

6. Convolamine, or a pharmacologically acceptable salt thereof, according to any of claims 1 to 4, for its use in the prevention or the treatment of an animal neuropathy, neurodegenerative pathology, neurodevelopmental pathology, psychiatric disorder or metabolic disorder.

7. Pharmaceutical composition comprising, or consisting of, as active principal ingredient, convolamine or a pharmacologically acceptable salt thereof, and an acceptable carrier, diluent or excipient, for its use in the prevention or the treatment of a condition or illness chosen among: a neuropathy, a neurodegenerative pathology, a neurodevelopmental pathology, a psychiatric disorder and a metabolic disorder.

8. Pharmaceutical composition according to claim 7, wherein convolamine or a pharmacologically acceptable salt thereof, is present in an amount of at least 80 % of the total dry weight of the composition.

9. Pharmaceutical composition according to claim 7 or 8, wherein said condition or illness is chosen among: Alzheimer's disease and anxiety.

10. Pharmaceutical composition according to any of claims 7 or 8, wherein the therapeutically effective amount of convolamine or a pharmacologically acceptable salt thereof, ranges from about 0.1 to about 10 mg/kg, when administered orally.
